(19) Europäisches Patentamt
European Patent Office
Office européen des brevets

(11) EP 1 184 455 A1

(12) # EUROPEAN PATENT APPLICATION
published in accordance with Art. 158(3) EPC

(43) Date of publication:
**06.03.2002 Bulletin 2002/10**

(21) Application number: **00935572.8**

(22) Date of filing: **07.06.2000**

(51) Int Cl.⁷: **C12N 15/09**, G06N 3/12

(86) International application number:
**PCT/JP00/03697**

(87) International publication number:
**WO 00/75301 (14.12.2000 Gazette 2000/50)**

(84) Designated Contracting States:
**AT BE CH CY DE DK ES FI FR GB GR IE IT LI LU MC NL PT SE**

(30) Priority: **08.06.1999 JP 16067299**

(71) Applicant: **Japan Science and Technology Corporation**
**Kawaguchi-shi, Saitama 332-0012 (JP)**

(72) Inventors:
- **KITANO, Hiroaki**
  **Kawagoe-shi, Saitama 350-0035 (JP)**
- **HAMAHASHI, Shugo, Sato Building 303**
  **Kawasaki-shi, Kanagawa 211-0025 (JP)**

(74) Representative: **Howden, Christopher Andrew**
**FORRESTER & BOEHMERT Pettenkoferstrasse 20-22**
**80336 München (DE)**

(54) **METHOD FOR PREDICTING BINDING SITE STRUCTURE OF GENE REGULATOR AND APPARATUS THEREFOR**

(57) A method for predicting the structure of a binding site to which a gene regulator binds, which method enables, through investigation of the internal structure of an enhancer or promoter, transcriptional regulation of a gene can be more clearly elucidated.

The method for predicting the gene regulator binding site structure includes providing a gene of interest, of which a user of the method desires to predict regulation-related structures of binding sites (12), (13), (14) to which regulators bind, the binding sites (12), (13), (14) being present within an enhancer or promoter (11) region to which a protein serving as a transcriptional element is bound and which is present upstream or downstream to a coding region (15) of the gene; constructing a calculation model for each of the binding sites (12), (13), (14) within the enhancer or promoter (11) region, the binding sites (12), (13), (14) being such that relevant regulators or hypothetically introduced regulators are to be bound thereto, the calculation model employing, as parameters, loci of the regulator binding sites or other factors that cause expression of the gene; computing the level of transcription of the gene with respect to the above-constructed calculation model; searching, through use of parameter search algorithms, parameters of the calculation model so that empirically known expression of the gene is obtained; to thereby predict microstructures of the enhancer or promoter.

FIG. 4

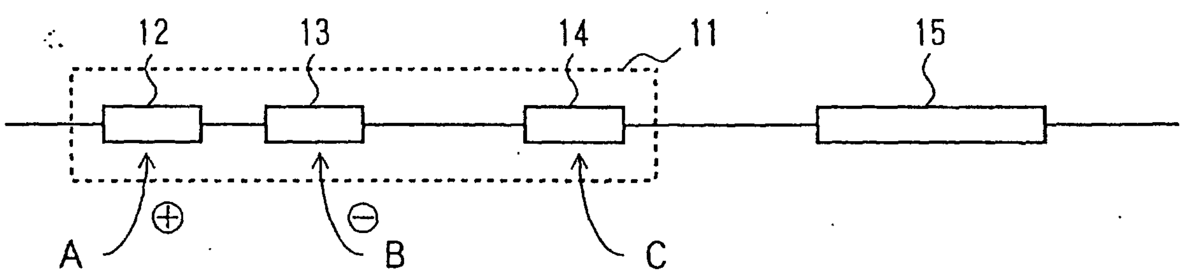




Printed by Jouve, 75001 PARIS (FR)

## Description

Technical Field

The present invention relates to regulation of a gene on the basis of, for example, expression data of the gene or concentration data of a protein with respect to the frame of space and time, and in particular to a method for predicting the structure of a binding site to which a gene regulator binds, as well as to an apparatus therefor.
The term "gene" denotes an essential unit of heredity, and encompasses mRNA (messenger ribonucleic acid).

Background Art

Background art will first be described while Drosophila, which is customarily used in molecular biological studies, is taken as an example.
In the first stage of development of Drosophila, at which point no cells have been formed, transcription products are spread by diffusion. Thus, the concentration of proteins that are present in the vicinity of a certain nucleus plays an important role in regulation of transcription. In simulation of this case, diffusion is calculated according to the following equation:

$$dU_i/dt = D_i \cdot (d^2U_i/dx^2)$$

wherein $U_i$ is the concentration of a protein i and $D_i$ is a diffusion coefficient of the protein i. Influence of the thus-diffused protein on expression of a gene is calculated by using a probability of binding to the corresponding binding region. For example, for the sake of simplicity, provided that proteins a and b compete for a single binding region, the probability at which the protein a attains binding is calculated as follows:

$$P = U_a/(U_a + U_b)$$

wherein $U_a$ and $U_b$ represent concentrations of proteins a and b, respectively.
However, in reality, binding affinity a must be considered, and therefore, P is expressed as follows.

$$P = \alpha_a \cdot U_a/(\alpha_a \cdot U_a + \alpha_b \cdot U_b)$$

Thus, conventional approaches to understanding of transcriptional regulation of a gene have relied only on protein concentration.
Fig. 1 is a schematic illustration showing conventional transcriptional regulation of a gene.
In Fig. 1, reference numeral 101 represents an enhancer for binding a protein and is located at an upstream region. The enhancer 101 functions as a promoter comprising a switch gene. Reference numeral 102 represents a coding region. Letters A and B represent proteins, and symbols + and - denote activation and repression, respectively.
As described above, transcriptional regulation of a typical gene is generally expressed by an equation in which only the concentration of protein i is employed.

Disclosure of the Invention

However, it has now become clear that the transcriptional regulation of a gene depends not only on the above-mentioned protein concentrations but also on the sites where the proteins are actually bound. Thus, elucidation of the internal structure of an enhancer or promoter has now become a very important subject.
In view of the foregoing, an object of the present invention is to provide, through investigation of the internal structure of an enhancer or promoter and with an aim toward further clarification of transcriptional regulation of a gene, a method for predicting the structure of a binding site to which a gene regulator binds, as well as an apparatus therefor.
To attain the above object, the present invention provides the following:

[1] A method for predicting the structure of a gene regulator binding site, comprising:

a step for providing a gene of interest, of which a user of the method desires to predict the regulation-related structure of a binding site to which a regulator binds, the binding site being present within an enhancer or

promoter region to which a protein serving as a transcriptional element is bound and which is present upstream or downstream to a coding region of the gene;
a step for constructing a calculation model for each of the binding sites within the enhancer or promoter region, the binding sites being such that relevant regulators or hypothetically introduced regulators are to be bound thereto, the calculation model employing, as parameters, loci of the regulator binding sites or other factors that cause expression of the gene;
a step for computing the level of transcription of the gene with respect to the above-constructed calculation model;
a step for searching, through use of parameter search algorithms, parameters of the calculation model so that empirically known expression of the gene is obtained; and
a step for predicting microstructures of the enhancer or promoter.

[2] The method for predicting the structure of a gene regulator binding site as described in [1], wherein said other factors that cause expression of the gene include temperature, molecular weight, diffusion coefficient, binding affinity, reaction rate, reverse reaction rate, and %transcription per occurrence of transcription.
[3] The method for predicting the structure of a gene regulator binding site as described in [1] or [2], wherein, from the searched parameter sets, microstructures comprising binding sites of the enhancer or promoter, some of the binding sites interacting locally with one another, are predicted as follows:

at portions where binding sites are dense, the microstructures are physically close to one another for interaction therebetween, or they are physically remote from one another but interact with one another closely and functionally, and
at portions where binding sites are sparse, the microstructures are physically remote from one another and yet functional, or they are functionally independent from one another.

[4] Apparatus for predicting the structure of a gene regulator binding site, comprising:

means for providing a gene of interest, of which a user of the method desires to predict the binding site to which a regulator binds, the binding site being present within an enhancer or promoter region to which a protein is bound and which is present in an upstream or downstream region;
means for constructing a calculation model which employs, as parameters, loci of the regulator binding sites within the enhancer or promoter region or other factors that cause expression of the gene;
means for computing the transcription level of the gene with respect to the above-constructed calculation model;
means for searching, through use of parameter search algorithms, parameters of the calculation model so that empirically known expression of the gene is obtained; and
means for predicting microstructures of the enhancer or promoter.

[5] The apparatus for predicting the structure of a gene regulator binding site as described in [4], wherein said other factors that cause expression of the gene include temperature, molecular weight, diffusion coefficient, binding affinity, reaction rate, reverse reaction rate, and %transcription per occurrence of transcription.

Brief Description of the Drawings

Fig. 1 is a schematic illustration showing conventional transcriptional regulation of a gene.
Fig. 2 is a block diagram in relation to an apparatus for predicting the structure of a gene regulator binding site according to an embodiment of the present invention.
Fig. 3 is a flow chart for predicting the structure of a gene regulator binding site and depicting an embodiment of the present invention.
Fig. 4 is a schematic diagram showing exemplary transcriptional regulation of a gene according to the present invention.
Fig. 5 is a conceptual sketch for explaining an enhancer or promoter region of eve2 in relation to an example of the present invention.
Fig. 6 is an enlarged view of the region A shown in Fig. 5.
Fig. 7 shows model structures of binding sites.
Fig. 8 is a graph showing the expression data of eve2.

Fig. 9 is a conceptual illustration of a solution space and solution candidates (candidate solutions) when solution sets are created at random.

Fig. 10 is a conceptual illustration of a solution space and solution candidates when solution sets are not created at random.

Fig. 11 is an illustration showing a solution 1.

Fig. 12 is an illustration showing a solution 2.

Fig. 13 is an illustration showing a solution 3.

Fig. 14 is an illustration showing the case where the binding sites are close to each other.

Fig. 15 is an illustration showing the case where the binding sites are remote from each other.

Best Mode for Carrying Out the Invention

Modes for carrying out the present invention will next be described with reference to the accompanying drawings.

Fig. 2 is a block diagram relating to an apparatus for predicting the structure of a binding site of a gene to which a regulator binds according to an embodiment of the present invention.

In Fig. 2, reference numeral 1 indicates an input unit; 2 a processing unit (CPU/memory); 3 a processing section for introducing a gene of interest, of which the user wishes to predict binding sites of regulators within an enhancer or promoter; 4 a processing section for constructing a calculation model in which loci of the binding sites of regulators within an enhancer or promoter are employed as parameters; 5 a processing section for calculating, on the basis of the constructed calculation model, the level of transcription; 6 a processing section for processing expression pattern data of the gene of interest; 7 a memory section for storing parameter search algorithms; 8 a calculation model parameter search processing section for searching, by use of parameter search algorithms, parameters usable in the calculation model so as to obtain an expression which is in agreement with empirically obtained expression of the aforementioned gene; 9 a processing section for predicting microstructures of an enhancer or promoter; and 10 an output unit.

Fig. 3 is a flow chart for predicting the binding site structure of a gene to which a regulator binds, and depicts an embodiment of the present invention, wherein

(1) First, a gene of interest is provided. The gene is such that there the user of the invention desires to predict the structure of a regulator binding site which is present within an enhancer or promoter which binds a protein and which is present upstream or downstream with respect to a coding region of the gene (Step S1).

(2) Next, a calculation model is constructed for each of the binding sites within the enhancer or promoter region, the binding sites being such that relevant regulators or hypothetically introduced regulators are to be bound thereto, the calculation model employing, as parameters, loci of the regulator binding sites (Step S2).

(3) Subsequently, the level of transcription of the gene is computed with respect to the above-constructed calculation model (Step 3).

(4) Then, through use of parameter search algorithms, parameters of the calculation model are searched so that empirically known expression of the gene is obtained (Step S4).

(5) Regarding Step S4, checking is performed as to whether parameters cannot be appropriately determined, or whether the obtained results are not good (Step S5).

(6) If the answer to the questions in Step S5 is YES, the constructed model is modified (Step S6).

(7) If the answer to the questions in Step S5 is NO, microstructures of the enhancer or promoter are conjectured (Step S7). Briefly, the microstructures of an enhancer or promoter can be predicted from the parameters obtained by use of search algorithms as follows: at a region where binding sites are dense, the microstructures predictable are such that binding sites are present physically close to one another and closely interact one another, or alternatively they are physically apart from one another but they interact functionally closely one another; and at a region where binding sites are sparse, they act on the gene independently, or in other words, the binding sites exist physically remote from one another, or their actions are functionally independent from one another.

In this connection, when all the regulators cannot be identified, unidentified regulators may be employed as parameters.

In addition, when all the binding sites are not identified (with respect to the distance and dimension), they may be treated as unidentified binding sites.

Hereafter, the method for predicting the structure of a gene regulator binding site will be described in detail.

Fig. 4 is a schematic diagram showing exemplary transcriptional regulation of a gene according to the present invention.

In Fig. 4, reference numeral 11 indicates an upstream-located enhancer, to which proteins A, B, and C are to

be bound. The enhancer 11 functions as a promoter formed of a switch gene. Here, the parameters which describe the enhancer — or promoter — 11 are loci (e.g., X-axis coordinates) of binding sites 12, 13, and 14 to which regulators bind, each site constituting a local structure of the enhancer. Reference numeral 15 indicates a coding region. Symbols + and - denote activation and repression, respectively.

As described above, a characteristic feature of the present invention resides in that the binding site structure of a given gene (including mRNA) is predicted on the basis of expression data of the gene. In other words, the invention enables inference of genetic microstructures (i.e., partial structure or sequence of an enhancer or promoter).

The method for predicting the structure of a binding site of a gene to which a regulator binds is generally described as follows.

(1) Generation of an initial population

A regulatory region of a gene is constructed such that the position of a binding site is used as a variable.

(2) Parameter estimation

For the initial population, parameters are introduced and estimated; the parameter estimation must be effected so as to determine parameters capable of generating expression data which approximate given expression data. The parameter estimation may be performed by use of, for example, a genetic algorithm or an annealing method.

(3) Microstructure estimation

If the above step for parameter estimation reveals, for example, that some regulator binding sites are identified at loci which are close to one another, an inference derived therefrom is that the regulators are also close to one another and interact locally.

On the other hand, if the binding sites are found to be present at loci which are remotely apart from one another, an inference derived therefrom is that the regulators independently and individually act on their respective binding sites.

Hereafter, the method for predicting the structure of a binding site to which a gene regulator binds will be described in detail with reference to Fig. 4.

(1) As shown in Fig. 4, local structures of an enhancer or promoter 11 are introduced (shown as regulator binding sites 12, 13, and 14).

Specifically, the loci corresponding to the respective regulator binding sites 12, 13, and 14 are employed as parameters.

(2) Parameter optimization

Through optimization of the location of the respective loci, there can be identified microstructures that cannot be functional unless they are close to — or conversely remote from — one another.

To this end, the following procedures are used.

1) Providing a gene of interest, of which the user desires to predict the structures (microstructures) within the enhancer or promoter 11.

2) Constructing a calculation model for each of the binding sites within the enhancer or promoter 11, the binding sites being such that relevant regulators or hypothetically introduced regulators are to be bound thereto. In the calculation model, loci of the regulators are employed as parameters.

3) Computing the level of transcription of the gene with respect to the above-constructed calculation model.

4) Using parameter search algorithms such as GA (genetic algorithm) and SA (simulated annealing), to search parameters of the calculation model so as to obtain empirically known expression of the gene provided in step 1).

5) From the resultant parameter sets, the following are inferred:

(i) At portions where binding sites are dense, microstructures are physically close to one another, or if they are physically remote from one another, they interact with one another closely and functionally.

(ii) At portions where binding sites are sparse, microstructures are physically remote from one another, or if they are physically close to one another, they are functionally independent from one another.

Next, the method for predicting the structure of a binding site to which a gene regulator binds will be described in more detail.

For illustration purposes, the following description is specifically directed to the case where the gene of interest is the second even-skipped gene (hereinafter referred to as eve2) of Drosophila, which gene relates to the formation of striped patterns.

Fig. 5 is a conceptual sketch for explaining an enhancer or promoter region of eve2 in relation to the exemplary case of the present invention.

In Fig. 5, B1 through B5 are binding sites for a bicoid gene (hereinafter referred to as bcd), H3 for a hunchback gene (hereinafter referred to as hb), K3 through K5 for a Kruppel gene (hereinafter referred to as Kr), and G1 through G3 for a giant gene (hereinafter referred to as gt). The binding sites indicated by circles relate to activation, and those indicated by squares relate to repression. When certain proteins called regulators bind to these binding sites, the enhancer or promoter activates or represses genetic transcription. Transcription of a gene is considered to be regulated through this mechanism.

As described above, according to most conventional theories, transcriptional regulation of a gene is elucidated through determinism on the basis of concentrations of the respective regulators concerned. For example, in the case of a model which depends on concentration alone, the probability of a transcription of a gene occurring is expressed by the following equation (1).

$$p = \frac{(\alpha_{B4}C_{B4} + \alpha_{B5}C_{B5})}{(\alpha_{B4}C_{B4} + \alpha_{B5}C_{B5}) + (\alpha_{K5}C_{K5} + \alpha_{G3}C_{G3})}$$

Now, in Fig. 5, attention is invited particularly to the area surrounded by broken lines (region A). Within this region, binding sites of a plurality of regulators are dense on the DNA sequence. Biological studies conducted in recent years have clarified that actual phenomena occurring within such a region A cannot be explained by simple reliance on the concentration of a regulator. In other words, within such a region A, binding and non-binding (which leads to transcriptional regulation of a gene) are determined not on the sole basis of the concentration of a regulator, but also on the basis of local competition in binding caused by high local density of the binding sites, which greatly affects binding and non binding.

Now, attention is focused on region A, and the phenomenon occurring in region A is described with reference to Fig. 6. A case is considered in which firstly Kr is bound to K5, as shown in Fig. 6. Originally, region B5 and region K5 share a common sequence. Under such conditions, one hypothesis is that, when binding occurs at either region, that region is entirely covered by the protein bound thereto, thereby nullifying the binding ability imparted to the binding site of the other region; i.e., the unbound region. Specifically, in the case shown in Fig. 6, K5 has been taken by Kr for binding thereto, and the sequence of B5 is "covered" by Kr and thus disabled. Similarly, even when a common sequence is not shared by two or more binding sites, depending on the size of a protein, a binding site other than the binding site available to that specific protein may also be covered., Also in such a case, the thus-covered binding site is disabled. In the particular case shown in Fig. 6, due to the binding of Kr having occurred, in effect, the other binding sites, B4, B5, and G3, are functionally disabled. As a result, all the activating binding sites present within the region A are hindered by Kr, and this explains why only repressing functions are exerted under such conditions. (Note that according to conventional theories which rely only on concentration, even under the above situation, if the concentration of bcd is high, activation-related function should occur, and this result does not agree with the actual situation.)

Thus, unlike the conventional approach in which concentration alone is used for describing a phenomenon as mentioned above, the method of the present invention, which takes into account not only concentration but also stochastic behavior at different points in time, enables determination of the level of transcription of the gene.

In connection with the aforementioned regulators of eve2, let us assume that the following are known: activating regulators are bcd and hb, and repressing regulators are Kr and gt (or that some of the regulators are unknown and hypothetical introduction thereof is permitted). Under such conditions and in the case where the structure of an enhancer or promoter of eve2 is unknown, or where presence of functional competition/non-competition is unknown, local structures of the regulators can be predicted by performing the below-described steps.

(1) Describing the structure of the enhancer or promoter

The region A in Fig. 5 is enlarged in Fig. 7, where a certain point X is established on the DNA sequence. The distance as counted on the DNA sequence from the point X to the binding site is introduced as a parameter. It is also possible to simultaneously introduce, to each of the binding sites, the size of the binding site itself, binding affinity (or binding probability, dissociation probability, etc.) of the binding site, level of contribution to transcription occurring when a regulator is bound to the binding site, and other possible variables which may be considered attributes of the binding site.

Specifically, the region A shown in Fig. 5 may be described as follows.

$$\text{Enhancer}\begin{cases} K5: & \text{distance } a & \text{size } l_a & \text{binding affinity } k_a & \cdots \\ B5: & \text{distance } b & \text{size } l_b & \text{binding affinity } k_b & \cdots \\ G3: & \text{distance } c & \text{size } l_c & \text{binding affinity } k_c & \cdots \\ B4: & \text{distance } d & \text{size } l_d & \text{binding affinity } k_d & \cdots \end{cases} \quad \text{......(2)}$$

Thus, the introduced binding sites are expressed by means of parameters. By changing the number of binding sites or values of these parameters, generally speaking, any type of enhancer structure or promoter structure can be adequately expressed.

(1) Experimental data

During the below-described "parameter search" procedure, experimental data are employed as solutions (also sometimes called optimal values or theoretical values). In the case of the present model, the experimental data are given as follows.

$$\text{Combination 1}\left\{\begin{matrix} bcd: & \alpha_{bcd} < C_{bcd} < \beta_{bcd} \\ hb: & \alpha_{hb} < C_{hb} < \beta_{hb} \\ Kr: & \alpha_{Kr} < C_{Kr} < \beta_{Kr} \\ gt: & \alpha_{gt} < C_{gt} < \beta_{gt} \end{matrix}\right\} \rightarrow \text{Transcription amount 1} \quad \text{......(3)}$$

$$\text{Combination 2}\left\{\begin{matrix} bcd: & \alpha_{bcd} < C_{bcd} < \beta_{bcd} \\ hb: & \alpha_{hb} < C_{hb} < \beta_{hb} \\ Kr: & \alpha_{Kr} < C_{Kr} < \beta_{Kr} \\ gt: & \alpha_{gt} < C_{gt} < \beta_{gt} \end{matrix}\right\} \rightarrow \text{Transcription Amount 2} \quad \text{......(4)}$$

$$\text{Combination } i\left\{\begin{matrix} bcd: & \alpha_{bcd} < C_{bcd} < \beta_{bcd} \\ hb: & \alpha_{hb} < C_{hb} < \beta_{hb} \\ Kr: & \alpha_{Kr} < C_{Kr} < \beta_{Kr} \\ gt: & \alpha_{gt} < C_{gt} < \beta_{gt} \end{matrix}\right\} \rightarrow \text{Transcription Amount } i \quad \text{......(5)}$$

Briefly, experimental data as to the transcription level of a gene are given in the form of combinations of regulator concentrations as described above. In reality, graphs as shown in Fig. 8 have already become readily available through experiments, and the profiles of the graphs are mathematically expressed by combinations as described above.

(3) Parameter search

When transcription of the enhancer or promoter expressed as described above is simulated, due to the presence of parameters whose values are not completely determined, the results of simulation may differ from those obtained through experiments. In such a case, through optimization of parameters by use of any appropriate means, parameters for the binding sites that had remained undetermined can be specifically determined.

Next will be described an example drawn to a case in which GA is employed as a parameter search algorithm which automatically determines parameters of a binding site by use of a computer.

[1] Generation of a population

First, a population consisting of a plurality (100, 1,000, or any other plural number) of solution candidates is generated. A solution set is introduced such that parameters are randomly distributed (see Fig. 9), or, for some reason, are converged to a local region of the entire solution space 21 (see Fig. 10). In Fig. 10, reference numeral 22 indicates a subspace in which solutions are conjectured to be present. For example, there may be a solution candidate having a distribution coefficient of 0, or another solution candidate having a distribution coefficient of 1.0. Similarly, there may be a solution candidate having a binding affinity of 0, or another solution candidate having a binding affinity of 0.01. Moreover, regarding the variables such as loci and other items, which have been introduced to the model and are now targets of search, values are changed to thereby provide a plurality of solution candidates.

$$\text{Solution Candidate 1} \begin{cases} K5 : Da_1 \ \ size\ l_{a1} \ \ BA\ k_{a1} \ \cdots \\ B5 : Db_1 \ \ size\ l_{b1} \ \ BA\ k_{b1} \ \cdots \\ G3 : Dc_1 \ \ size\ l_{c1} \ \ BA\ k_{c1} \ \cdots \\ B4 : Dd_1 \ \ size\ l_{d1} \ \ BA\ k_{d1} \ \cdots \end{cases} \qquad \ldots\ldots(6)$$

$$\text{Solution Candidate 2} \begin{cases} K5 : Da_2 \ \ size\ l_{a2} \ \ BA\ k_{a2} \ \cdots \\ B5 : Db_2 \ \ size\ l_{b2} \ \ BA\ k_{b2} \ \cdots \\ G3 : Dc_2 \ \ size\ l_{c2} \ \ BA\ k_{c2} \ \cdots \\ B4 : Dd_2 \ \ size\ l_{d2} \ \ BA\ k_{d2} \ \cdots \end{cases} \qquad \ldots\ldots(7)$$

$$\text{Solution candidate } i \begin{cases} K5 : Da_i \ \ size\ l_{ai} \ \ BA\ k_{ai} \ \cdots \\ B5 : Db_i \ \ size\ l_{bi} \ \ BA\ k_{bi} \ \cdots \\ G3 : Dc_i \ \ size\ l_{ci} \ \ BA\ k_{ci} \ \cdots \\ B4 : Dd_i \ \ size\ l_{di} \ \ BA\ k_{di} \ \cdots \end{cases} \qquad \ldots\ldots(8)$$

The above exemplifies actual solution candidates. Each of these solution candidates describes one enhancer or promoter. Therefore, it will be understood that solution candidates of different parameter values provide different transcription levels.

[2] Measurement of transcription level

Since the created solution candidates contain a variety of values of variables, the candidates exhibit different transcription levels when tested under identical conditions. Thus, under certain common test environments, the transcription level is measured for each of the solution candidates.

In this step, there are solution candidates which, under a specific test environment, fit— or do not fit—experimental values.

[3] Comparison between simulation results and experimental data

Assuming that under a certain test environment we have information about the transcription level of the gene being handled, the transcription level simulated under the same test environment is compared with the experimentally obtained value. For example, when the transcription level of a gene X is empirically found to be T at a concentration of a regulator A of $C_A$, comparison is performed at a fixed concentration $C_A$ of the regulator A by simulating the transcription level of the gene X and determining the difference between T and the value from the simulation. In simple cases, differences are often represented by absolute values of subtraction (but not necessarily always).

$$\triangle = |T_{simulated} - T_{found}| \qquad (9)$$

In this case, the smaller the value of $\Delta$, the more approximate the simulation to the experimental results. Thus, it can be decided that the solution candidate which can generate a transcription level more approximate to the experimental result is superior to other solution candidates, in order to obtain a transcription level similar to that obtained by experiments.

[4] Artificial selection / natural selection / genetic manipulation

Through comparison with experimental data, the appropriateness of the solution candidate can be assessed. The appropriateness is represented by a "fitness value," and in genetic algorithms, the fitness value represents the viability of the solution candidate. While solution candidates with excellent fitness values can retry transcription in a subsequent assessment, those with poor fitness values cannot.

In fact, this is the process of artificial selection and natural selection. According to genetic algorithms, after assessment of solution candidates, those with favorable traits are selected, and among the thus-selected candidates assessment is performed again. However, repetition of this process results in reduction in solution candidates, and therefore, replication is performed so as to make the number of candidates constant. Through this process, solution candidates with poor assessment ratings are not selected; and solution candidates which are not selected in subsequent assessment steps are thus screened out.

In genetic manipulations, mutation and cross-over are effected. Specifically, when mutation occurs, a previous diffusion coefficient of 0.5, for example, is changed to 0.6 through a certain value determination method (in some cases randomly changed and in other cases changed through use of a method such as SA). Also, "cross-over" is referred to as a process of selecting a solution candidate A and solution candidate B, and portions of them are mutually exchanged.

Thus through repetition of assessment of fitness values and artificial selection/natural selection/genetic manipulation, the entirety of the solution candidates are gradually refined.

With certain minor errors being ignored, there can be obtained a solution candidate group which has been optimized—or semi-optimized—through an optimization algorithm (note: in the case of optimization being imperfect, the situation may be referred to as "semi-optimized"). From the solution candidate members of the group, actual enhancer or promoter structures can be predicted.

The solutions can be expressed as follows:

$$\text{Optimal Solution } i \begin{cases} K5: & Da_{opt\ i} & \text{size } l_{a\ opt\ i} & BA\ k_{a\ opt\ i} & \cdots \\ B5: & Db_{opt\ i} & \text{size } l_{b\ opt\ i} & BA\ k_{b\ opt\ i} & \cdots \\ G3: & Dc_{opt\ i} & \text{size } l_{c\ opt\ i} & BA\ k_{c\ opt\ i} & \cdots \\ B4: & Dd_{opt\ i} & \text{size } l_{d\ opt\ i} & BA\ k_{d\ opt\ i} & \cdots \end{cases} \quad \ldots\ldots(10)$$

wherein D = distance, and BA = binding affinity.

Taking the region A in Fig. 5 as an example, optimization performed as described above may provide optimal solutions as shown in Figs. 11, 12, or 13.

That is, in the case of Fig. 11, a solution candidate provides a situation similar to that shown in region A in Fig. 5.

Alternatively, as in the case of Fig. 12, another solution candidate may provide a similar situation with K5 and G3 being exchanged.

In the case of Fig. 13, yet another solution candidate may provide a situation in which the binding sites are located at discrete positions (loci).

Thus, some converged solution candidates can be obtained.

[5] When solutions are obtained:-

With respect to these solutions, there can be obtained specific values for the enhancer or promoter structures which are desirable for yielding the results mimicking those obtained through experiments. As a result, the following can be detected, speculated upon, or concluded.

(a) In the case where binding sites are densely located, as shown in Fig. 14, since the binding sites are close to each other, these binding sites interact locally during transcription of a gene.

i. There exists a requirement that they must be close to each other as physical loci.
ii. As physical loci they may be remote from each other, but due to their close mutual interaction in terms of function, the solutions are close to each other.

(b) In the case where binding sites are sparsely located, as shown in Fig. 15, since the binding sites are present remote from each other, they independently act on genes during transcription thereof.

i. There exists a requirement that they must be present remote from each other as physical loci.
ii. As physical loci they may be close to each other, but due to their independent functionality, the solutions as obtained result.

Thus, without any information about the structure of an enhancer or promoter, through simultaneous optimization of values related to binding affinity, reaction rate, reverse reaction rate, etc. of the binding sites, and the situation of interaction of the binding sites contained in the enhancer or promoter, it is possible to know, before actual values are in hand, values which are likely to be found in experiments.

Being constituted as described above, the present invention exerts the following effects.

In the manufacture of chemical substances or pharmaceuticals where the influence of a certain substance on a gene is desired to be investigated in order to know, for example, whether the substance activates or represses the gene, let us assume that the efficacy of the substance acting on the gene is unknown due to the structure of enhancer or promoter of the gene being not completely known. Or alternatively, let us assume that, although speculation is that transcription of the gene is effected under competition between that particular substance and other substances, this speculation has not yet been verified.

Under such circumstances, the present invention enables very prompt and low-cost prediction regarding the efficacy of the substance with respect to its action on the gene, or regarding local interaction of the substance with other substances.

Conventional methods involve drawbacks in that thorough checking of the sequence of target enhancer or promoter structure is required, and the action of unknown substances cannot be taken into account at all.

However, according to the present invention, since the internal structures of an enhancer or promoter are considered, transcriptional regulation of a gene can be more clearly elucidated. The microstructures of an enhancer

or promoter can be predicted from the parameters searched as follows: at a region where binding sites are dense, the loci are present in the vicinity or the binding sites closely interact with one another; and at a region where binding sites are sparse, they act on the gene independently.

Therefore, in cases as described above, the present invention may be implemented as follows: Firstly, computer-aided simulation is performed for prediction, and thereafter, suitable biological experiments are designed and carried out. This process alleviates labor, time, and costs, which have heretofore been a significant burden because of the conventional approaches being performed on a trial and error basis. Moreover, in the pharmaceutical or like industry, prediction of the side effects of a drug can be realized.

The present invention is not limited to the above-described embodiments. Numerous modifications and variations of the present invention are possible in light of the spirit of the present invention, and they are not excluded from the scope of the present invention.

As described hereinabove in detail, according to the present invention, the following effects are obtained.

(A) Through investigation of the internal structure of an enhancer or promoter, transcriptional regulation of a gene can be more definitely elucidated.
(B) From the parameter set obtained through searching, the microstructures of the enhancer or promoter are predicted such that, in a region where binding sites are dense, the binding sites constitute local microstructures, and in a region where binding sites are sparse, they act on the gene independently.
(C) Thus, in the manufacture of chemical substances or pharmaceuticals, experiments which have conventionally been performed on a trial and error basis, requiring long periods of time, can be performed promptly and accurately.

Industrial Applicability

According to the present invention, experiments with respect to regulation of a gene, which have conventionally been performed on a trial and error basis requiring long periods of time can be performed promptly and accurately, and thus the invention is useful in the manufacture of chemical substances and pharmaceuticals.

## Claims

**1.** A method for predicting the structure of a gene regulator binding site, comprising:

(a) a step for providing a gene of interest, of which a user of the method desires to predict the regulation-related structure of a binding site to which a regulator binds, the binding site being present within an enhancer or promoter region to which a protein serving as a transcriptional element is bound and which is present upstream or downstream to a coding region of the gene;
(b) a step for constructing a calculation model for each of the binding sites within the enhancer or promoter region, the binding sites being such that relevant regulators or hypothetically introduced regulators are to be bound thereto, the calculation model employing, as parameters, loci of the regulator binding sites or other factors that cause expression of the gene;
(c) a step for computing the level of transcription of the gene with respect to the above-constructed calculation model;
(d) a step for searching, through use of parameter search algorithms, parameters of the calculation model so that empirically known expression of the gene is obtained; and
(e) a step for predicting microstructures of the enhancer or promoter.

**2.** The method for predicting the structure of a gene regulator binding site as described in claim 1, wherein said other factors that cause expression of the gene include temperature, molecular weight, diffusion coefficient, binding affinity, reaction rate, reverse reaction rate, and %transcription per occurrence of transcription.

**3.** The method for predicting the structure of a gene regulator binding site as described in claim 1 or 2, wherein, from the searched parameter sets, microstructures comprising binding sites of the enhancer or promoter, some of the binding sites interacting locally with one another, are predicted as follows:

at portions where binding sites are dense, the microstructures are physically close to one another for interaction therebetween, or they are physically remote from one another but interact with one another closely and functionally, and
at portions where binding sites are sparse, the microstructures are physically remote from one another and

yet functional, or they are functionally independent from one another.

**4.** Apparatus for predicting the structure of a gene regulator binding site, comprising:

(a) means for providing a gene of interest, of which a user of the method desires to predict the binding site to which a regulator binds, the binding site being present within an enhancer or promoter region to which a protein is bound and which is present in an upstream or downstream region;
(b) means for constructing a calculation model which employs, as parameters, loci of the regulator binding sites within the enhancer or promoter region or other factors that cause expression of the gene;
(c) means for computing the transcription level of the gene with respect to the above-constructed calculation model;
(d) means for searching, through use of parameter search algorithms, parameters of the calculation model so that empirically known expression of the gene is obtained; and
(e) means for predicting microstructures of the enhancer or promoter.

**5.** The apparatus for predicting the structure of a gene regulator binding site as described in claim 4, wherein said other factors that cause expression of the gene include temperature, molecular weight, diffusion coefficient, binding affinity, reaction rate, reverse reaction rate, and %transcription per occurrence of transcription.

F I G. 1

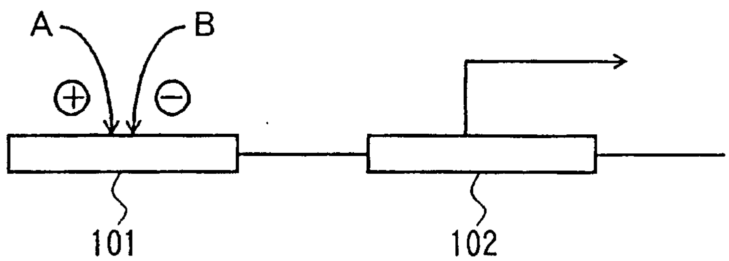

F I G. 2

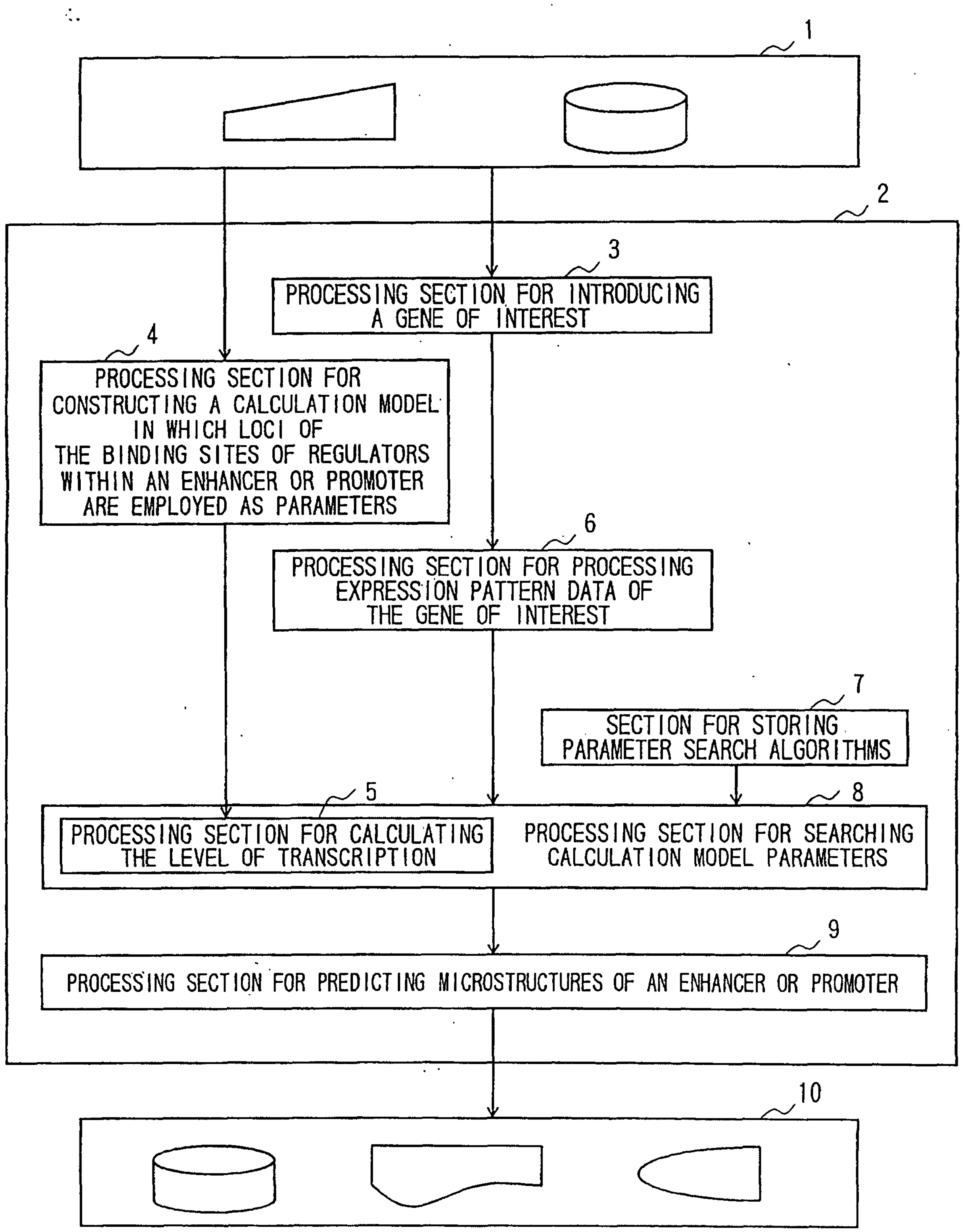
1
2
3
PROCESSING SECTION FOR INTRODUCING A GENE OF INTEREST
4
PROCESSING SECTION FOR CONSTRUCTING A CALCULATION MODEL IN WHICH LOCI OF THE BINDING SITES OF REGULATORS WITHIN AN ENHANCER OR PROMOTER ARE EMPLOYED AS PARAMETERS
6
PROCESSING SECTION FOR PROCESSING EXPRESSION PATTERN DATA OF THE GENE OF INTEREST
7
SECTION FOR STORING PARAMETER SEARCH ALGORITHMS
5
PROCESSING SECTION FOR CALCULATING THE LEVEL OF TRANSCRIPTION
8
PROCESSING SECTION FOR SEARCHING CALCULATION MODEL PARAMETERS
9
PROCESSING SECTION FOR PREDICTING MICROSTRUCTURES OF AN ENHANCER OR PROMOTER
10

FIG. 3

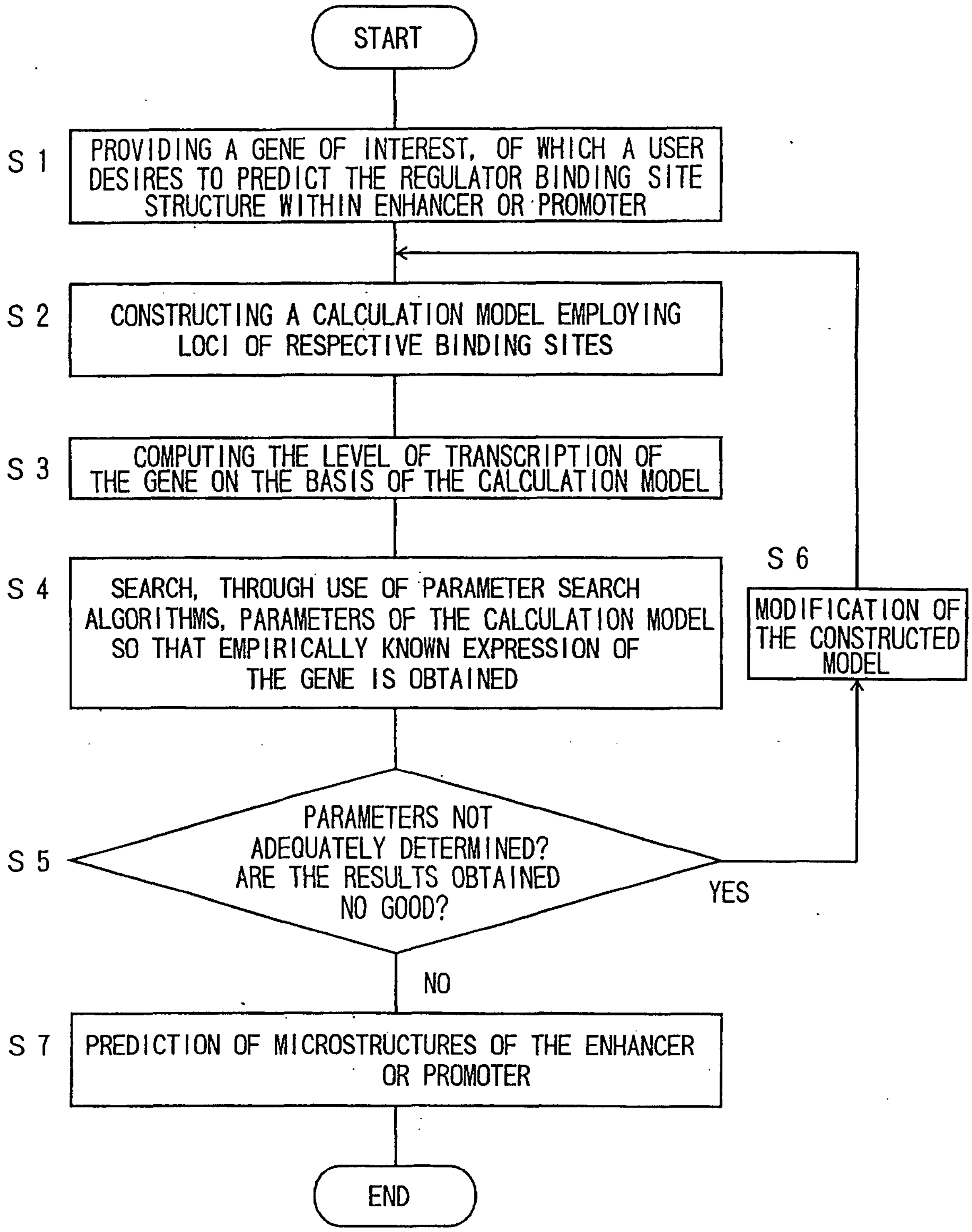
START
S 1
PROVIDING A GENE OF INTEREST, OF WHICH A USER DESIRES TO PREDICT THE REGULATOR BINDING SITE STRUCTURE WITHIN ENHANCER OR PROMOTER
S 2
CONSTRUCTING A CALCULATION MODEL EMPLOYING LOCI OF RESPECTIVE BINDING SITES
S 3
COMPUTING THE LEVEL OF TRANSCRIPTION OF THE GENE ON THE BASIS OF THE CALCULATION MODEL
S 4
SEARCH, THROUGH USE OF PARAMETER SEARCH ALGORITHMS, PARAMETERS OF THE CALCULATION MODEL SO THAT EMPIRICALLY KNOWN EXPRESSION OF THE GENE IS OBTAINED
S 5
PARAMETERS NOT ADEQUATELY DETERMINED? ARE THE RESULTS OBTAINED NO GOOD?
YES
S 6
MODIFICATION OF THE CONSTRUCTED MODEL
NO
S 7
PREDICTION OF MICROSTRUCTURES OF THE ENHANCER OR PROMOTER
END

FIG. 4

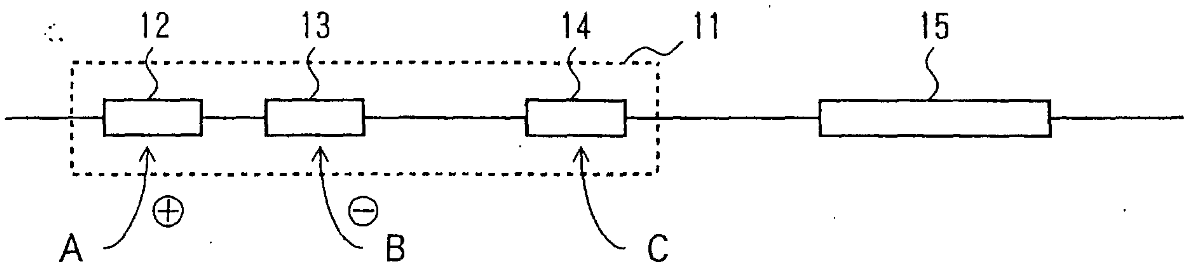


FIG. 5

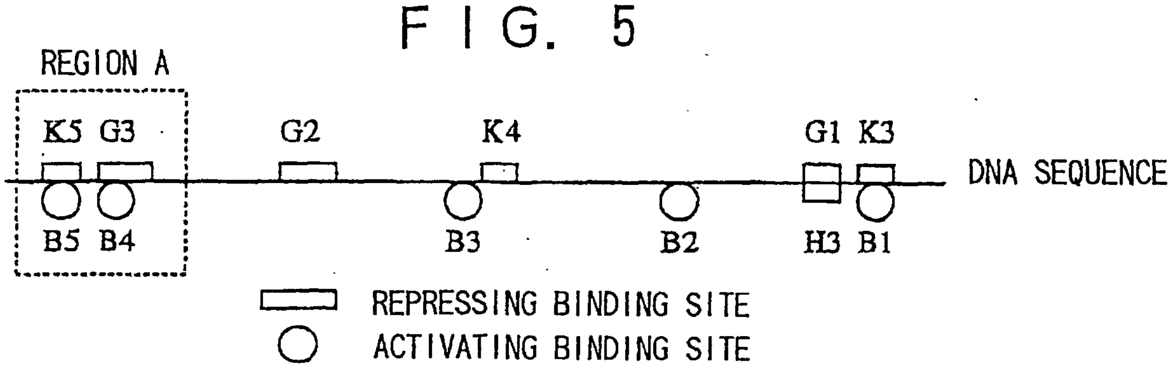


FIG. 6

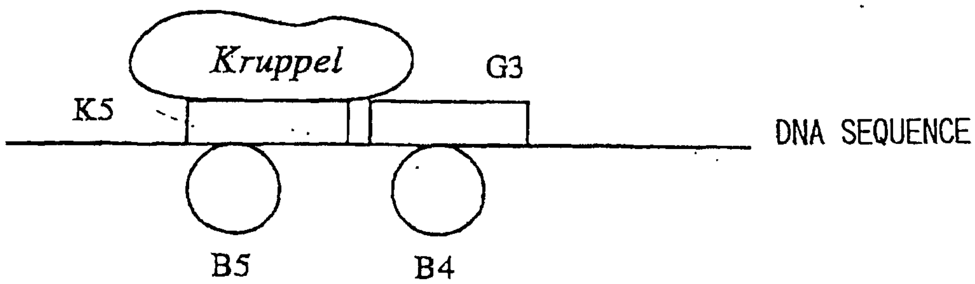

F I G. 7

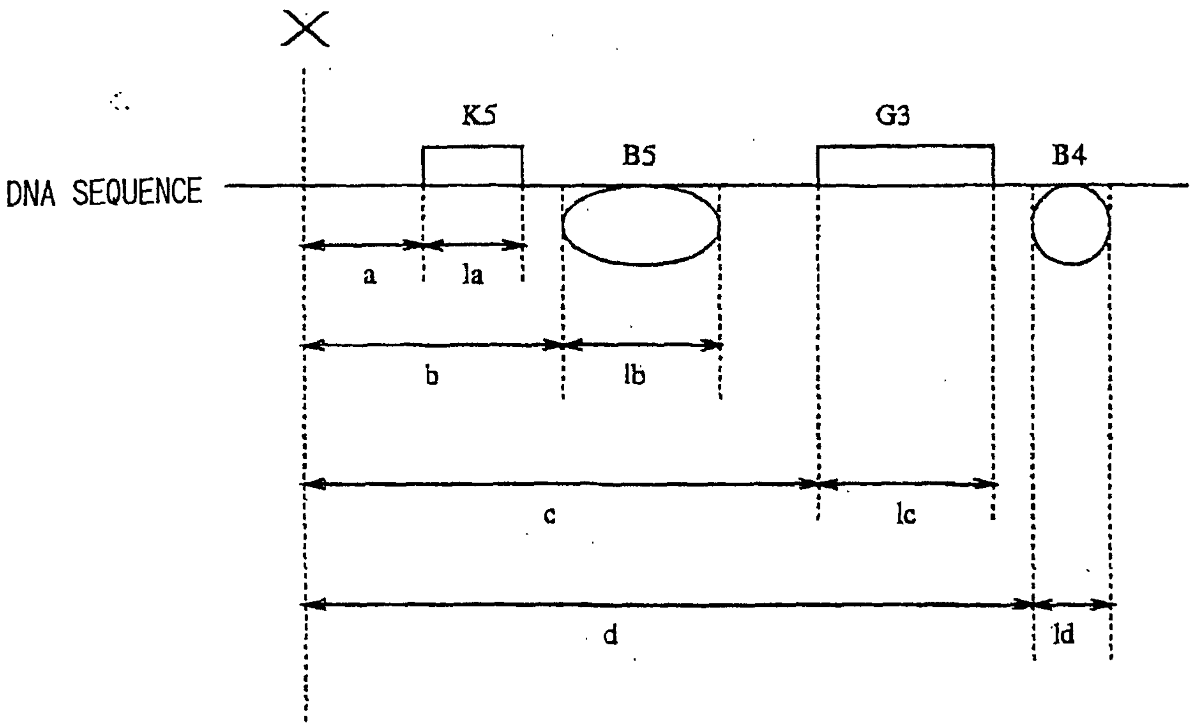
X
K5
B5
G3
B4
DNA SEQUENCE
a
la
b
lb
c
lc
d
ld

F I G. 8

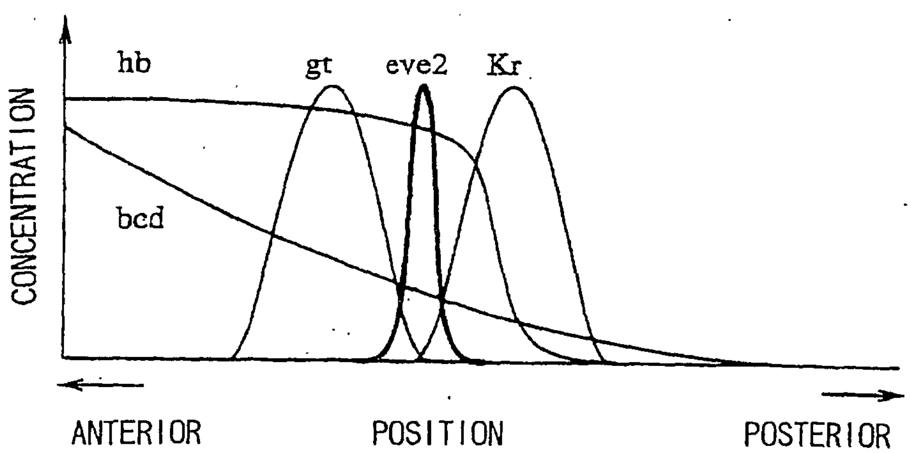
hb
gt
eve2
Kr
bcd
CONCENTRATION
ANTERIOR
POSITION
POSTERIOR

FIG. 9

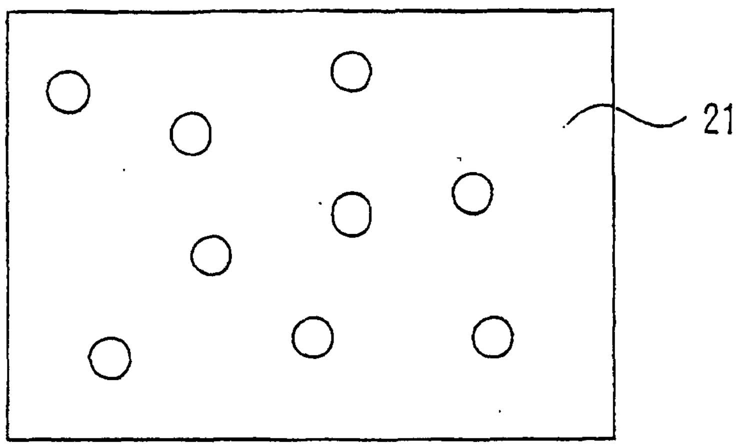


FIG. 10

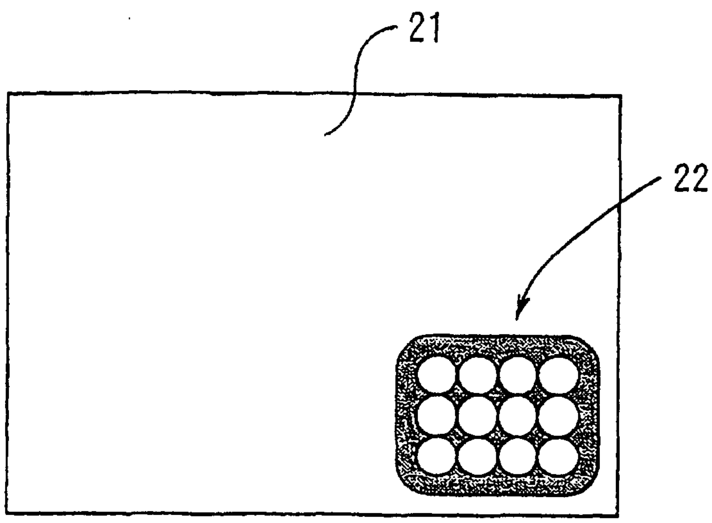


FIG. 11

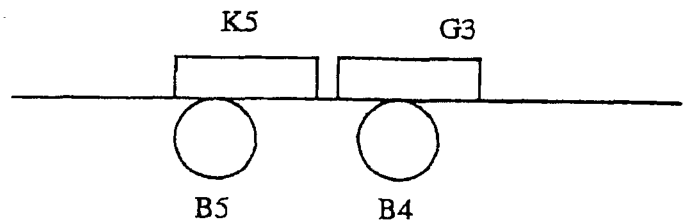

FIG. 12

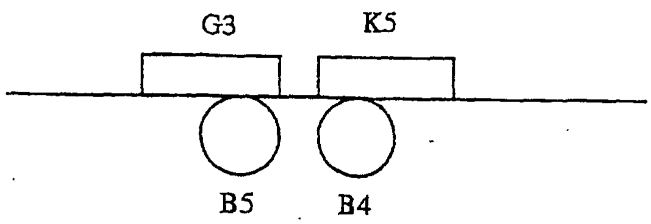


FIG. 13

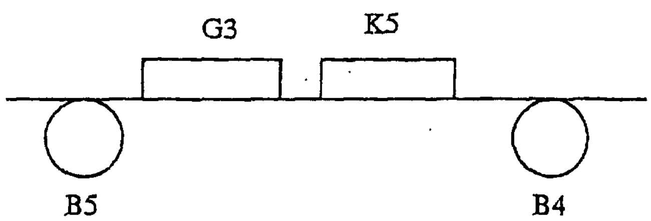


FIG. 14

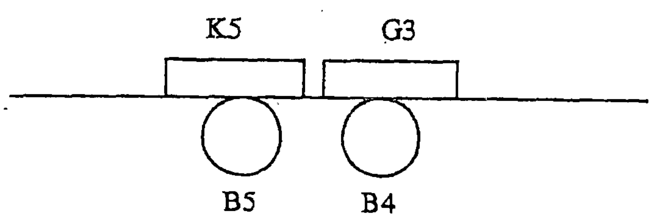


FIG. 15

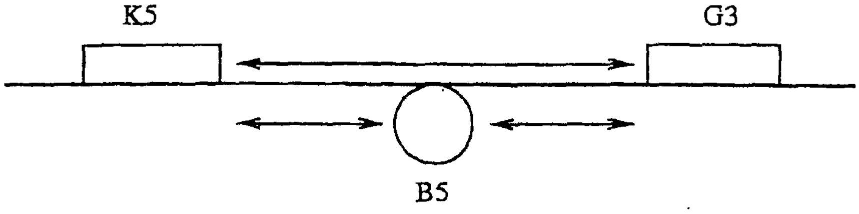

## INTERNATIONAL SEARCH REPORT

International application No.
PCT/JP00/03697

A. CLASSIFICATION OF SUBJECT MATTER
Int.Cl[7] C12N15/09, G06N3/12

According to International Patent Classification (IPC) or to both national classification and IPC

B. FIELDS SEARCHED
Minimum documentation searched (classification system followed by classification symbols)
Int.Cl[7] C12N15/09, G06N3/12

Documentation searched other than minimum documentation to the extent that such documents are included in the fields searched

Electronic data base consulted during the international search (name of data base and, where practicable, search terms used)
WPI(DIALOG), BIOSIS(DIALOG)

C. DOCUMENTS CONSIDERED TO BE RELEVANT

| Category* | Citation of document, with indication, where appropriate, of the relevant passages | Relevant to claim No. |
|---|---|---|
| PA | WO, 2000/111145, A1 (Fujitsu Ltd.)<br>02 March, 2000 (02.03.00)<br>& JP, 2000-60553, A | 1-5 |
| PA | JP, 2000-57124, A (NEC Corporation et al.)<br>25 February, 2000 (25.02.00)<br>(Family: none) | 1-5 |
| A | US, 5598350, A1 (National Institute of Genetics, et al.)<br>28 January, 1997 (28.01.97)<br>& GB, 2283840, A & JP, 7-274965, A | 1-5 |
| A | JP, 6-261755, A (Toshiba Corp.)<br>20 September, 1994 (20.09.94)<br>(Family: none) | 1-5 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 August, 2000 (04.08.00) | 19 September, 2000 (19.09.00) |
| Name and mailing address of the ISA/<br>Japanese Patent Office<br><br>Facsimile No. | Authorized officer<br><br>Telephone No. |

Form PCT/ISA/210 (second sheet) (July 1992)